(19) Europäisches Patentamt
European Patent Office
Office européen des brevets

(11) Publication number: 0 551 062 A2

# (12) EUROPEAN PATENT APPLICATION

(21) Application number: 92850308.5

(22) Date of filing: 22.12.92

(51) Int. Cl.[5]: C08L 23/16, C08J 3/28, C08F 8/00, C08K 5/00, A61L 31/00

(30) Priority: 10.01.92 FI 920116

(43) Date of publication of application: 14.07.93 Bulletin 93/28

(84) Designated Contracting States: AT BE CH DE DK ES FR GB GR IE IT LI LU MC NL PT SE

(71) Applicant: NESTE OY
P.O.Box 310
SF-06101 Porvoo(FI)

(72) Inventor: Matthijs, Dirk
Sacramentstraat 5 c
B-9910 Knesselare(BE)

(74) Representative: Örtenblad, Bertil Tore
Noréns Patentbyra AB Box 27034
S-102 51 Stockholm (SE)

## (54) Polymer composition which withstands sterilization carried out by irradiation, and a method for the production thereof.

(57) The invention relates to a method for producing a polymer composition which withstands sterilization carried out by irradiation, and to a polymer composition produced by the method. According to the invention, the composition is made up of an ethylene-propylene copolymer in the polymer frame of which the proportion of ethylene is approx. 1-6 % and which contains as additives, calculated from the amount of the copolymer: LD polyethylene having a density of 0,915-0,930 g/cm$^3$, approx. 0,1-5 %; HALS-type amine, approx. 0,05-0,3 %; dibenzylidene sorbitol, which may be alkyl-substituted, approx. 0,01-5 %; organic phosphite, approx. 0,01-0,5 %; and inorganic stearate, approx. 0,01-1 %. The said additives are added in one step to the said ethylene-propylene copolymer, which is preferably in the form of a power and is thereafter molded into sterilizable products, particularly various hospital and laboratory equipment.



Rank Xerox (UK) Business Services
(3. 10/3.6/3.3. 1)

The invention relates to a polymer composition which withstands sterilization carried out by irradiation, and to a method for producing such a polymer composition.

Polymer material which withstands sterilization treatment is needed especially for hospital and laboratory equipment, such as single-use injection syringes, catheters, test tubes, and surgical clamps. To avoid the risk of infection, such products must be absolutely clean and sterile before use, and this is best achieved by sterilizing the final products in their packages.

Said polymer products have been sterilized by treating them with gases or ethylene oxide. However, irradiation of such products with high-energy radiation, such as gamma rays, beta rays or X-rays, is becoming an increasingly popular sterilization method. Irradiation is especially well suited for the sterilization treatment of products which are in packages.

Polypropylenes constitute a suitable material for the said hospital and laboratory equipment. The advantages of polypropylene include light weight, transparency, good processability, chemical resistance, and a suitable combination of ductility and impact strength. There is, however, the disadvantage that the high-energy radiation used for sterilization makes polypropylene brittle and turns it yellow. The brittleness is caused by breakage of polymer chains, and the yellowing is caused by the phenolic antioxidants used as stabilizers.

According to EP patent publication 7736, the yellowing of a polyolefin, such as polypropylene, by gamma radiation has been reduced by adding to the material certain HALS-type (Hindered Amine Light Stabiliser) amines. However, the materials disclosed in the publication involve the problem that their properties deteriorate with time for the reason that an effective antioxidant system cannot be used.

The object of the present invention is to provide a new polymer composition which withstands sterilization by irradiation without coloration while substantially retaining its physical properties both in connection with the irradiation and during storage of the product thereafter. The polymer composition according to the invention is characterized in that it is made up of an ethylene-propylene copolymer in the polymer backbone of which the proportion of ethylene is approx. 1-6 % and which contains as additives, calculated from the amount of the copolymer,

- LD polyethylene having a density of 0.915-0.930 g/cm$^3$ approx. 0.1-5 %,
- HALS-type amine approx. 0.05-0.3 %,
- unsubstituted or alkyl-substituted dibenzylidene sorbitol approx. 0.01-0.5 %,
- organic phosphite approx. 0.01-0.5 %, and
- inorganic stearate approx. 0.01-1 %.

According to the invention, the polymer composition is produced substantially by mixing the additives listed above into the said ethylene-propylene copolymer in one step. Preferably the mixing takes place into a copolymer in powder form.

A polymer having the composition according to the invention is transparent, withstands, without noteworthy yellowing, gamma radiation of 5 Mrad, and retains well its physical properties. The organic phosphite serves as a stabilizer in the polymer composition, but the improvement achieved with the invention is evidently based on a synergic combined effect of the phosphite and of the LD polyethylene and HALS amine present in suitable amounts.

The principal component of the polymer composition according to the invention is made up of an ethylene-propylene copolymer, in the backbone of which ethylene, in a proportion of approximately 1-6 %, is randomly distributed. The melt flow rate of the copolymer at 230 °C and with a load of 2.16 kilograms may vary within the range 0.4-50 g/10 min.

The LD polyethylene with a density of 0.915-0.930 g/cm$^3$, belonging to the composition according to the invention, for its part contributes to the stabilization of the composition during the irradiation. It is known that LD polyethylene itself withstands high-energy radiation, and in addition it forms crosslinks which make the polymer more ductile and improve its chemical resistance.

The HALS-type amine included in the composition is known to act as a radiation-withstanding stabilizer. The amine may be, for example, a 2,2,6,6-tetramethyl-piperidine derivative having the formula

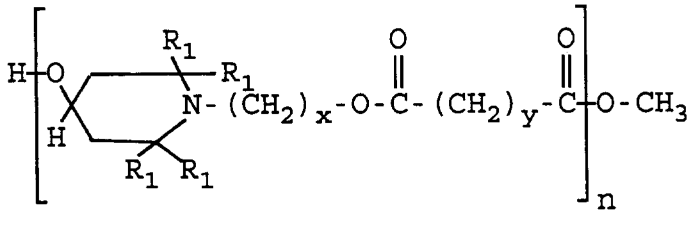

where $R_1$ is an alkyl group containing 1-3 carbon atoms, x and y are integers of 2-4, and n is an integer of 5-20. One example of a suitable compound is poly-(N-β-hydroxyethyl-2,2,6,6,-tetramethyl-4-hydroxy-piperidyl succinate), which is marketed under the name Tinuwin$^R$622LD.

Another example of the said HALS-type amines is a compound marketed under the name Chimassorb$^R$944LD and having the formula

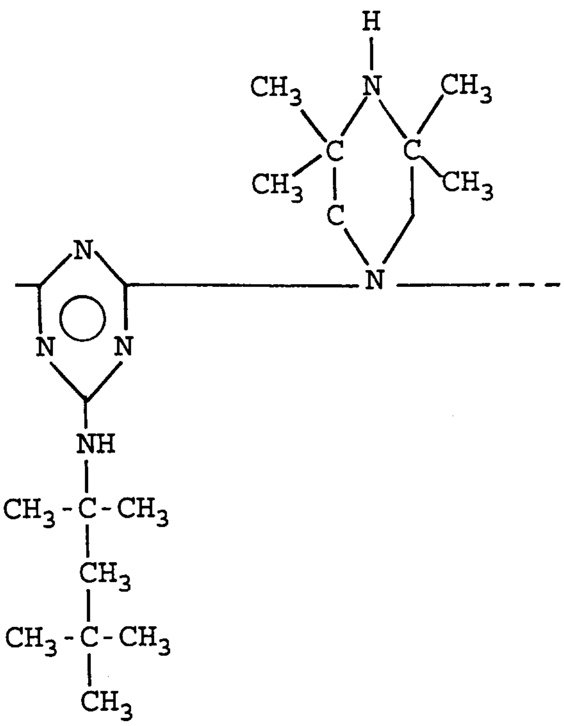

The dibenzylidene sorbitol belonging to the composition and having the formula

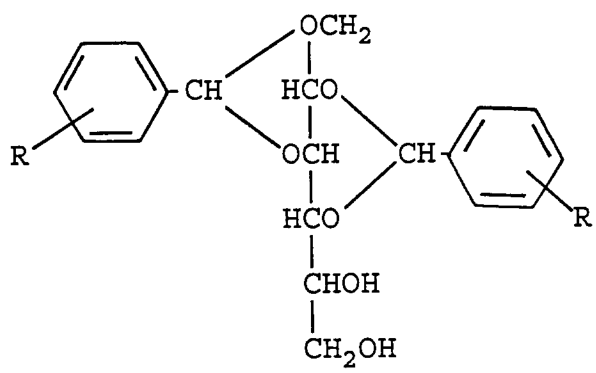

where R is hydrogen or an alkyl group containing 1-18 carbon atoms, serves as a nucleator for the ethylene-propylene copolymer. The dibenzylidene sorbitol improves the rigidity, impact strength and transparency of the polymer material. The crystallization temperature of the polymer also rises, which speeds up the solidification of the polymer. At the same time the produced spherulites decrease in size, which in turn improves the ability of the polymer to withstand high-energy radiation.

The purpose of the organic phosphite belonging to the composition is to serve as a stabilizer in the mixing phase of the ethylene-propylene copolymer and the additives to be added to it and in the subsequent processing of the polymer composition into a completed product. Furthermore, the phosphite protects the dibenzylidene sorbitol used as the nucleator. An example of suitable phosphites is phenol-2,4-bis(1,1-dimethylethyl)phosphite sold under the name Irgafos 168® and having the formula

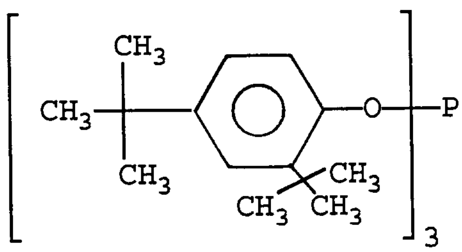

The purpose of the stearate belonging to the composition, e.g. calcium stearate, is to neutralize any Ziegler-Natta catalyst residues, in particular chlorides.

Polymer products are manufactured from a polymer composition according to the invention preferably so that the additives, i.e. LD polyethylene, HALS amine, dibenzylidene sorbitol, organic phosphite, and stearate, are mixed in one step into powdered ethylene-propylene copolymer, whereafter the mixture is brought into a molten state, in which it is molded, for example, by injection molding or by blow molding, into the final shape required of the products. The final products are packaged and thereafter sterilized by, for example, iradiation with gamma rays.

Example

The following components (percentages calculated from the amount of copolymer) were added to an ethylene-propylene copolymer which contained ethylene 3 %, randomly distributed into the polymer backbone, and the MFR value of which at 230 °C and with a load of 2.16 kilograms was 20 g/10 min:

a) calcium stearate 0.1 %,

b) phenol-2,4-bis(1,1-dimethylethyl)phosphite (Irgafos 168$^R$) 0.1 %,

c) poly-(N-$\beta$-hydroxyethyl-2,2,6,6-tetramethyl-4-hydroxypiperidyl succinate) (Tinuwin$^R$622LD) 0.15 %,

d) methyldibenzylidene sorbitol 0.2 %, and

e) LD polyethylene having a density of 0.918 g/cm$^3$ and a melt index of 7 g/10 min at 190 °C and with a 2.16 kilogram load 2 %.

After the different components had been combined, test pieces were molded of the polymer composition for the testing of coloration and tensile strength, as well as for the determination of hardness (Charpy test). The test pieces were irradiated with a gamma ray dose of 3.8 Mrad obtained from a $Co^{60}$ source, whereafter they were aged at 80 °C in a recirculated air oven. The results obtained are shown in the following table.

| | | Aged at 80 °C (weeks) | | | | | | | | | |
|---|---|---|---|---|---|---|---|---|---|---|---|
| | | 0 | 1 | 2 | 3 | 4 | 5 | 6 | 7 | 8 | 9 |
| Yellowing index (2-mm sheets) | 4.1 | 5.4 | 6.9 | 6.5 | 6.8 | 7 | 7.4 | 7.6 | 7.8 | 8.3 | 9.1 |
| Charpy-test on a notched sheet, 23 °C | 7.6 | 6.8 | 7.1 | 7.2 | 7.2 | 7.2 | 7.2 | 6.5 | 7.7 | 7.3 | 7.2 |
| Tensile stress at yield point 23 °C | 26.7 | 28.3 | 30.5 | 30.2 | 30 | 29.6 | 27.1 | 29.5 | 25.0 | 29.3 | 27.1 |
| Tensile stress at breaking point | - | 30.1 | 31.1 | 30.6 | 29.5 | 29.7 | 26.6 | 29.3 | 25.3 | 29.2 | 26.3 |
| Elongation at yield point | 11.5 | 10.9 | 10.8 | 10 | 6.9 | 8.1 | 7.5 | 3.9 | 5.7 | 8.2 | 6.1 |

The results indicate that no significant coloration occurs in the polymer material as a consequence of irradiation, and also that the physical properties of the material are not significantly weakened. The material thus withstands very well high-energy radiation sterilization and is a suitable material especially for the sterile hospital and laboratory equipment mentioned above.

## Claims

1. A method for producing a polymer composition which withstands sterilization carried out by irradiation, **characterized** in that into an ethylene-propylene copolymer, in the polymer backbone of which the proportion of ethylene is approx. 1-6 %, there are mixed as additives in one step, calculated from the amount of the copolymer
   - LD polyethylene having a density of 0.915-0.930 $g/cm^3$ approx. 0.1-5 %,
   - HALS-type amine approx. 0.05-0.3 %,
   - unsubstituted or alkyl-substituted dibenzylidene sorbitol approx. 0.01-0.5 %,
   - organic phosphite approx. 0.01-0.5 %, and
   - inorganic stearate approx. 0.01-1 %.

2. A method according to Claim 1, **characterized** in that the amine is a 2,2,6,6-tetramethyl-piperidine derivative, such as poly-(N-$\beta$-hydroxyethyl-2,2,6,6-tetramethyl-4-hydroxy-piperidyl succinate).

3. A method according to Claim 1 or 2, **characterized** in that the organic phosphite is phenol-2,4-bis(1,1-dimethylethyl) phosphite.

4. A method according to any of Claims 1-3, **characterized** in that the stearate is calcium stearate.

5. A method according to any of the above claims, **characterized** in that the additives are mixed into a copolymer which is in powder form.

6. A method according to Claim 5, **characterized** in that the powder is brought into a molten state, in which it is molded into polymer products which are sterilizable by irradiation with gamma rays, beta rays or X-rays.

7. A polymer composition withstanding sterilization by irradiation, produced by the method according to any of the above claims, **characterized** in that it is made up of an ethylene-propylene copolymer in the polymer backbone of which the proportion of ethylene is approx. 1-6 % and which contains as additives, calculated from the amount of the copolymer
   - LD polyethylene having a density of 0.915-0.930 $g/cm^3$ approx. 0.1-5 %,
   - HALS-type amine approx. 0.05-0.5 %,
   - unsubstituted or alkyl-substituted dibenzylidene sorbitol approx. 0.01-0.5 %,
   - organic phosphite approx. 0.01-0.5 %, and
   - inorganic stearate approx. 0.01-1 %.

8. A composition according to Claim 7, **characterized** in that the composition is in the form of a powder.

9. A composition according to Claim 7, **characterized** in that the composition is incorporated into a molded polymer product which is sterilizable by irradiation with gamma rays, beta rays or X-rays.